# EUROPEAN PATENT SPECIFICATION

(11) **EP 0 865 300 B1**
(45) Date of publication and mention of the grant of the patent: **15.09.2004**
(21) Application number: 96944265.6
(22) Date of filing: 06.12.1996
(51) Int. Cl.: A61M 5/00

(54) **A DEVICE FOR APPLYING A MIXTURE OF TWO LIQUID COMPONENTS**
VORRICHTUNG ZUM VERABREICHEN EINER MISCHUNG AUS ZWEI FLÜSSIGKOMPONENTEN
APPAREIL POUR L'APPLICATION D'UN MELANGE DE DEUX COMPOSANTS LIQUIDES

(30) Priority: 07.12.1995 US 8237 P
(43) Date of publication of application: 23.09.1998
(73) Proprietor: BRISTOL-MYERS SQUIBB COMPANY, Skillman, NJ 08558 (US)
(72) Inventor: HOLM, Niels, Erik, DK-3460 Birkerod (DK); GARBASCH, Allan, DK-3460 Birkerod (DK); FAIRBROTHER, John, E., Flintshire CH7 6EA (GB); CASTELLANA, Frank, Skillman, NJ 08558 (US)
(74) Representative: Mays, Julie
(86) International application number: PCT/US1996/019505
(87) International publication number: WO 1997/020585

(56) References cited:
- EP-A- 0 634 140
- EP-A- 0 669 100
- DE-A- 4 223 356
- DE-U- 29 603 183
- US-A- 4 846 405

## Description

### Technical Field

The present invention relates to a device for co-applying two or more liquid components from separate containers via separate liquid channels or tubes having separate outlets. In preferred embodiments the present device is useful for co-applying fibrin sealant components with a gas to form a spray.

### Background Art

US-PS No.4,359,049 to Redl discloses a double barrel syringe for applying a tissue adhesive such as fibrin glue or fibrin sealant to a human or animal in need thereof. The fibrin sealant described comprises predominantly two major components, a fibrinogen-containing component and a thrombin-containing component, each in liquid form upon use. Essentially, the thrombin and fibrinogen, when mixed, provide that the peptide chains of the fibrinogen are cleaved and conditions are provided so that the resulting fibrin polymerizes into a clot which is useful for sealing fluid and air leaks, in haemostasis and to connect tissue. To avoid premature clot formation double-barrelled applicators are employed which, of course, keep the two components separate until application to a patient is required. The '049 patent discloses that pistons within the two cartridges, each containing one component, can be commonly actuated to dispense fluid simultaneously from each.

Other prior art patents describe various mixing heads for mixing two or more components used in these and other surgical sealants. For example, US-PS No. 5,116,315 assigned to Hemaldics describes a head where the liquid conducts leading from the component cartridge enter a mixing chamber fashioned so as to provide a swirling of the components before they exit a common exit channel. Adequate mixing of the components is desired so as to form a uniform fibrin sealant. Inefficient mixing results in the coadministration of fibrinogen and thrombin which may only result in a small yield of actual sealant. A difficulty with fibrin sealant applicators can be the premature formation of the clot within the device, especially those devices where the components are mixed within a mixing head and/or those devices wherein the components exit through a common channel. After the first spray of sealant is complete, a clot may block the exit channels rendering the applicator useless and greatly reducing the surgeon's flexibility in carrying out the sealant part of the surgical procedure.

US-PS No. 4,631,055 to Immuno includes a gas conveying channel for blowing a gas through the needle or mixing head during discharge of the components. However, an even, uniform distribution of the materials over the anatomical area of interest is still not achieved. Indeed, a significant amount of the components are wasted.

EP-PS No. 592,242 to Edwardson et al. discloses the first completely autologous fibrin sealant. It provides for the coadministration of a fibrin monomer solution with a buffer solution conducive to polymerisation of the fibrin monomer prepared in less than 30 minutes from a single source of blood (preferably that of the patient to receive the sealant). This breakthrough technology provides a fixed amount of fibrin monomer solution from a sample of about 140 to 160 ml of blood. Uniform and efficient mixing is even more important in order to benefit from this safe, efficient, autologous sealant product and therefore new devices and methods for applying two or more components to form a surgical sealant would be useful addition to the art.

PCT Application WO 96/17618 discloses a device and a method of applying components of a fibrin sealant. The device comprises a source of a gas and a reservoir for each component wherein the gas source and component reservoirs can be actuated by common means. Each of the reservoirs and gas source are separately in fluid communication with a spray head having a center aperture at its outlet end and annular apertures, arranged concentric with and radially outward from the centre aperture whereby the gas and each of said components are discharged through separate apertures. Preferably, the gas is discharged through the centre aperture and the fibrin sealant forming components are discharged separately through each of the annular apertures.

A device according to the preamble of claim 1 is disclosed in document DE 42 23 356.

### Brief Description of the Invention

In accordance with the present invention there is disclosed a device for utilizing a gas to co-apply two or more liquid components with enhanced uniformity of mixing of the components resulting in a more homogeneous mixture of the components and a minimal amount of unutilized components. The present device comprises a nozzle head having outlets out of which the liquid components and gas are dispensed and where the outlets are arranged in a straight line with the gas outlet at one end of that line. Also disclosed is a device for applying two or more liquid components, e.g., components which form a fibrin sealant, wherein the sources of said components, and preferably the source of a gas to be used, are in fluid communication with an applicator and are remote from that applicator.

In a described method of question of the device of the present invention, the flow rates of the components and gas are selected such that the two liquid components are drawn or sucked into the gas being expelled from the nozzle head as they exit their respective outlets of the nozzle head.

In this manner the two or more components are mixed outside the nozzle before they are sucked into the gas jet since the component exiting the outlet farthest from the gas outlet sucked across the outlet nearer to the gas outlet. The components are further mixed in the gas jet and the mixture and gas form a spray. In addition, the outlets are relatively small with the result that a relatively small amount of the components is exposed to the surroundings when the expelling is stopped. Furthermore, the clogging tendency in the outlets is substantially eliminated since there is no mixing of components in the nozzle head. Also the amount of the components remaining in said outlets after the application procedure is almost negligible. The latter factor is of vital importance in connection with a mixture of an autologous fibrin monomer solution and a buffer solution where the amount of fibrin monomer solution is relatively limited and accordingly must be utilized to an optimum.

When the components are discharged at mutually differing volume flows, it is particularly advantageous to discharge the component with the lowest volume flow through the outlet accommodated farthest away from the gas jet. In this manner it is ensured that the component being discharged with the lowest volume flow is mixed with the component being discharged with the highest volume flow before said components enter the gas jet in the mixed state.

The expelling of the components through the nozzle head may according to be carried out by means of an expelling means, which is activated by means of an electronic control unit, and the expelling of the gas may be carried out by means of a pump also activated by means of the electronic control unit, said electronic unit in turn being activated by means of a signaller controlled by the user handling the nozzle head. As a result, the expelling of the components and the gas can be carried out in a homogeneous manner at substantially constant volume flows.

In connection with a method using syringes with their respective expelling piston as containers for the components, the interruption of the expelling means through the control unit may imply that the control unit activates said expelling means to retract the pistons of the syringes a short distance with the result that the components are retracted a short distance from the outlets of the nozzle. As a result, the risk of the two components coming into contact with one another and consequently the risk of a clogging of the nozzle outlets have been considerably reduced.

The invention relates furthermore to a device for applying a mixture of two liquid components kept in their respective containers, where said components are fed to a nozzle head through their respective liquid-transferring means, said nozzle head comprising a separate outlet channel for each component as well as a separate outlet channel for a gas fed through a gas-transferring means from a gas source, and where said device comprises an expelling means simultaneously activating the containers containing their respective component. The component outlets and the gas outlet in the nozzle head are aligned along a substantially rectilinear line, the gas outlet being accommodated at one end of the line of outlets. Such a nozzle head is relatively easy to manufacture by way of any convenient means, e.g., injection moulding, and furthermore it ensures an efficient, good mixture of the components.

According to the invention the nozzle head may be supported by an elongated handle, and the component and gas-transferring means may be substantially resilient tubings which extend from said handle to a remote accommodation of the expelling means with the containers and the gas source, respectively. The resulting handling of the nozzle head is relatively easy and can be carried out without interference by the presence of the expelling means and the gas source.

Furthermore, the handle may according to the invention be associated with a push-button for actuation of the expelling means and the feeding of gas through a differential pressure-adjusting device. As a result, the component source containers, e.g., the pistons in the syringe cartridges, can be actuated to supply the gas and components without the presence of electricity adjacent the nozzle head and the associated handle.

When the containers used for the components are syringes with their respective piston for expelling the component in question, the expelling means may according to the invention comprise a holder for the securing of the syringes, and an electrically driven elevator means for a simultaneous actuation of the pistons of the syringes with the result that a particularly accurate and constant volume flow set in advance is obtained through each outlet in the nozzle upon such actuation.

Finally, it is according to the invention particularly preferred that the expelling means and the gas source in form of a gas pump are combined as a separate unit together with an electrically driven control unit, said control unit being adapted to control the expelling means and the gas pump at a signal from the user of the nozzle head.

### Brief Description of the Drawings

The invention is explained in greater detail below with reference to the accompanying drawing, in which
Fig. 1 shows a device according to the invention,
Fig. 2 is a front view of a nozzle head used in the device, and
Fig. 3 the same taken along the lines I-I in Fig. 2.
Fig. 4 shows another view of a device according to the invention.

### Description of the Preferred Embodiments

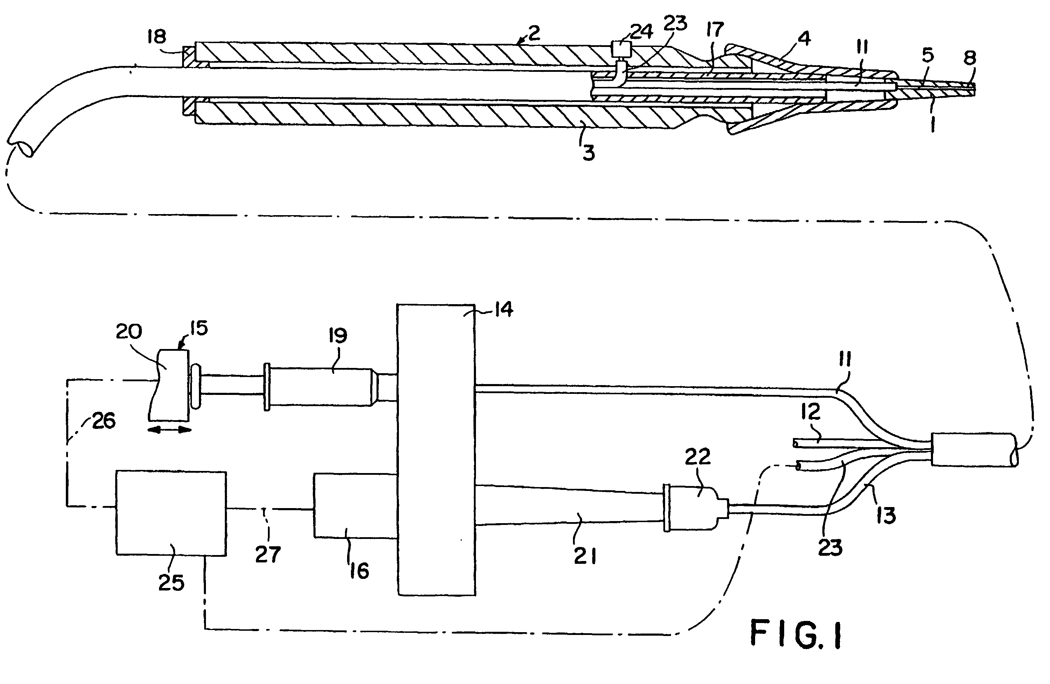

The device shown in Fig. 1 comprises a nozzle head 1 secured at the end of a handle designated the general reference numeral 2. This handle comprises a tubular body 3 connected through a transition portion 4 to the nozzle head 1, said tubular body 3, said transition portion 4, and said nozzle head 1, respectively, having a circular cross section. However, any convenient handle or means to hold or accommodate the nozzle head could be employed.

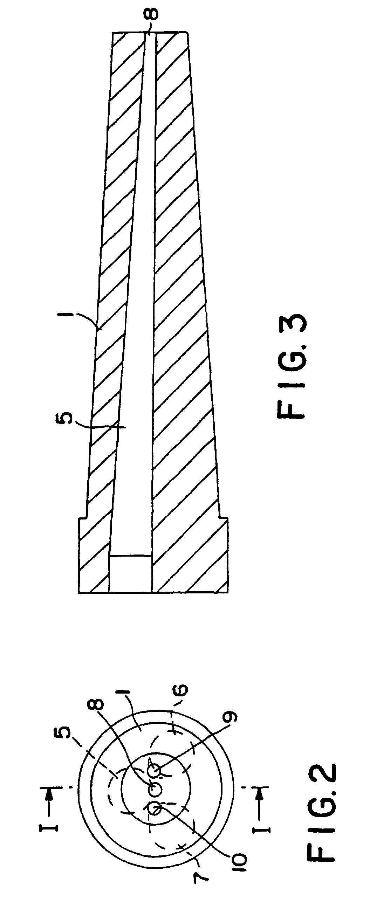

As illustrated in particular in Figs. 2 and 3, the nozzle head is preferably conically shaped with three through channels 5, 6, and 7, where two channels serve as outlet channels for two different components while the third channel serves as outlet channel for a gas. Of course, additional channels and outlets can be utilized as required by the particular material or product to be applied. The outlet channels 5, 6, and 7 are formed by any convenient means, e.g., by the nozzle head being molded in a suitable plastic material, such as polystyrene material, SAN. The outlet channels extend from their respective opening at the broadest end of the nozzle head 5 adjacent the handle 2, said openings being placed at the corners of an isosceles triangle, cf. the dotted lines of Fig. 2. At the corners of the isosceles triangle, each channel is of a diameter of approximately 1.2 mm, and preferably after a short distance with a substantially constant diameter each channel narrows down so as to be of the desired diameter at the opposite end. This is done to accommodate using fluid transfer tubing which may be larger in diameter than the desired outlet size. Any convenient diameter outlets can be utilized, but outlets having diameters of .3 to .5 mm and preferably .3mm are preferred. At this end of the channels, the outlets are aligned along a rectilinear line with a center distance of approximately 0.5 mm. The outlets have been designated the reference numerals 8, 9, and 10, respectively, in Fig. 2.

The above short distance of a substantially constant inner diameter of the outlet channels 5, 6 and 7 in the nozzle head serves to receive the end of fluid transfer tubes 11, 12, and 13, respectively, which in turn are provided for the feeding of said components and said gas. These fluid transfer tubes 11, 12, and 13 extend axially through the handle 2 and forwards to a coupling plate 14, which is arranged and associated with an expelling means for the components and designated the general reference numeral 15, as well as a gas pump 16. The tubings 11-15 can be surrounded by a resilient protecting jacket 17. At the end of the handle 2 opposite the nozzle head 1, the protecting jacket 17 with the tubings 11-12 can be preferably retained in the handle 2 by means of a cap 18 inserted thereon. The cap 18 is provided with a central, through opening and co-operates with the protecting jacket 17 and is retained in said handle 2 by way of friction.

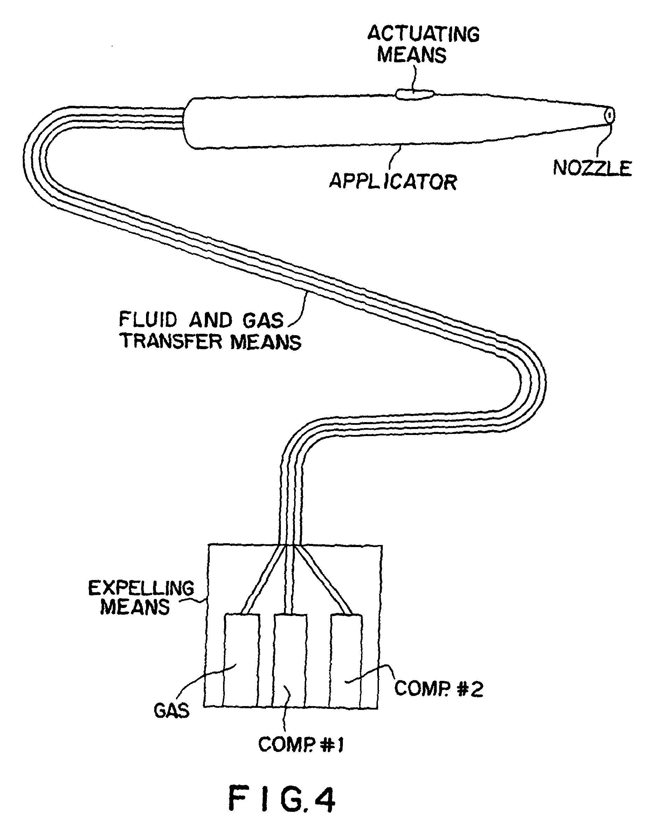

As mentioned above, the preferred device according to this invention comprises a spray applicator or "pen" which is remote from, but in fluid communication with, the sources of liquid components and/or gas. In areas such as the application of fibrin sealants this provides a greatly improved tool for the surgeon. This is because the prior art fibrin sealant applicators all include the sources of components in the hand-held applicator. As discussed previously, prior art fibrin sealant applicators are typically double-barrel syringes attached to a Y-piece or mixing lead. This is generally an awkward instrument for the surgeon to control and even when the surgeon uses both hands, a less than desirable application is achieved. One embodiment of the device of this invention is set forth in Figure 4.

The present spray applicator comprises a nozzle head with outlets and separate fluid transfer tubes for each component providing fluid communication between the outlets and the sources of components to be applied. The nozzle head has a handle which can be an elongated "pen" accommodating the nozzle head at one end with the fluid transfer tubes extending therethrough, exiting the opposite end and continuing to said sources of components. The sources of components at the remote location, which can be in any convenient containers, e.g., syringe cartridges, are preferably part of an expelling means which dispenses or expels these components from the sources through the fluid transfer tubes and out of the outlets of the nozzle head, as desired by the operator or surgeon. This can be accomplished by any convenient means such as valves in the nozzle head or handle of the applicator or preferably by providing an actuating means which sends a signal to the supply unit upon actuation by the operator or surgeon. The actuating means can be a foot pedal or a button in the applicator as are known in the device art. In a preferred embodiment the actuating means is a resilient bladder positioned in the applicator and connected via a pressurized line to a signal means, such that upon depression of the resilient bladder by the fingertip or hand of the surgeon the signal means detects a difference in pressure and signals the supply unit to supply the components.

The gas used to co-apply the components can be also supplied from, and controlled by, the expellingness in a preferred embodiment or, can be supplied and/or controlled separately from the liquid components.

Returning to Figure 1 in further describing the preferred expelling means, at the coupling plate 14, the fluid transfer tubes 11, 12 are inserted in their respective through bore (not shown), at the opposite end of which the tubings are adapted to be connected with their respective syringe 19 (or other convenient component source container) for their respective component. The drawing shows only one syringe 19 communicating with the tubing 11. The coupling plate 14 is adapted such that both the tubings 11 and 12 and the syringes, such as the syringe 19, can be easily removed. Beyond the latter coupling plate 14 for receiving the syringes 19, the expelling means 15 comprises an elevator means 20 for expelling the contents of the syringes 10.

As illustrated in Fig. 1, the third tubing 13 serves to feed the gas and is connected to the gas pump 16 through an outlet nozzle 21 and a luer coupling 22. The luer coupling 22 implies furthermore that the above tubing can be easily coupled to and removed from said coupling plate 14. A filter means is provided within the outlet nozzle 21.

As illustrated in Fig. 1, a fourth tubing 23 is furthermore accommodated inside the protecting jacket 17. The said fourth tubing 23 is at one end associated with a diagrammatically shown push-button 24 inside the handle 2, while the opposite end thereof is detachably connected to a control unit 25 in a manner not described in greater detail, said control unit being associated with the expelling means 15. When the push-button is pressed down, the tubing 23 with said push-button 24 and the control unit 25 is adapted to transfer the resulting pressure difference inside the tubing 23 as a signal to the control unit 25. As a result, the control unit initiates through the diagrammatically shown tubings 26, 27 the necessary activation of the elevator means 20 of the expelling means 15 and the air pump 16, respectively. The tubing 23 is detachably coupled to the control unit 25 in a manner not described in greater detail by means known to those skilled in the art.

The described device is mainly intended for application of a mixture of two or more components in, e.g., fibrin monomer and a buffer fluid with a pH of 10, respectively, as set forth in the aforementioned WO 96/17638 and EP 592,242. These two components are received in their respective syringe, such as the syringe 19, said syringes being dimensioned such relative to one another that the desired mixing ratio is established by a simultaneous activation of the pistons of the syringes by means of the elevator means 20. The mixing ratio of the pH 10-buffer to fibrin monomer is preferably 1 to 7, and usually a 5 ml syringe is used for receiving fibrin monomer and a 1 ml syringe for the pH 10-buffer. The tubings 11-13 are according to a preferred embodiment shaped by way of co-extrusion, the inner layer of the tubings being made of polyurethane which is inert to the fluids in question, and the outer layer of said tubings being made of polyurethane or PVC. The tubings are formed with an inner diameter of 0.3 mm with the result that a length of 2 m only allows approximately 0.3 ml of fluid to be received in each tubing, i.e., a total of .6 ml of fluids.

When the expelling means 15 and the air pump 16 are activated by the push-button 24 being pressed down, the two components are expelled from their respective outlet in the nozzle head 1 while the gas is simultaneously being expelled through the associated outlet in the nozzle head.

The gas is expelled through the outlet at one end of the row of outlets, while the component to be fed in the lowest amount, viz. the pH-buffer, is expelled through the outlet 9 provided at the opposite end. The other component, fibrin monomer, is expelled through the outlet 8 in the middle of the row of outlets. These components can be expelled at any convenient rate but low flow rates have been found to be advantageous. Accordingly, the pH 10 buffer can be expelled at flow rates between .0040 and .0100 ml/sec and the fibrin solution between .028 and .070 ml/secs preferably at the aforementioned 1:7 ratio. Most preferably, the fibrin monomer and pH 10-buffer to be mixed are expelled at a volume flow of approximately 0.04 ml/sec and 0.0057 ml/sec, respectively.

Air is used as gas in the preferred embodiment, said air being sucked in from the surroundings and subsequently carried from the pump 16 through a filter to the nozzle head 1. Alternatively, any source of desired gas can be employed. The speed of the air molecules must be relatively high at the same time as the amount of air used must be kept at a minimum. An outlet diameter of 0.3 mm has the effect that the volume flow of the air is approximately 1.25 l/min. during the expelling procedure, lower volume flow being preferred.

When the air and the two components flow out of their respective outlets, the fluids are sucked into the flow of air and are substantially atomized. The mixing of the two fluid components has been at least partially accomplished when said components are sucked into the flow of air. A lowering of the respective discharge speeds is carried out by an adjustment of the diameters of the outlets and their mutually distance in such a manner that both are as small as possible. In this manner it is possible to operate with relatively long tubings, i.e. the distance between the nozzle head 1 and the coupling plate 14 can be relatively long, such as for instance 2 m. The low discharge speeds render it possible to apply the liquid mixture in thin uniform layers with the result that a layer of a higher total thickness may be provided by laying more layers on top of each other.

The expelling of the components from the respective syringes is as men-tioned carried out inside the expelling means 15 which forms a combined unit together with the control unit 25. Both the expelling means 15 and the control unit 25 are electrically driven. This unit may be placed relatively far away from the site where the mixture is to be applied, and it can be adapted for reuse, whereas the nozzle head with the handle 2 and tubings 11, 12, and 13 may be adapted to be disposable. The control unit 25 can be such that an interruption of the application procedure before the syringes 19 are empty can have the effect that the elevator means 20 retracts the pistons in the syringes 19 a short distance in such a manner that the fluids in question are sucked a short distance away from the outlets in the nozzle head 1. In this manner it is avoided that an interruption of the applying procedure involves a post-expelling caused by pressures accumulated in the walls of the tubings, and especially in optionally present air bubbles in the syringe containing fibrin I. The preferred retraction volume is approximately 0.04 ml, which corresponds to the expelling for 1 sec.

The described device renders it possible to apply a fluid mixture in a thickness of approximately 170 µm, which implies that an amount of 5 ml fibrin and a corresponding amount of pH 10-buffer can cover an area of 300 cm². In connection with a length of tubing of approximately 2 m, the total amount of lost fluid in said tubings is, as mentioned, approximately 0.3 ml, whereby the amount of not used components after the complete expelling from the syringe is relatively low.

The invention has been described with reference to a preferred embodiment of a device for dispensing a mixture of fibrin monomer and a pH 10-buffer. The mixing ratios used must ensure that the mixture is relatively neutral, and preferably said neutral state has already been achieved in the air before the application site has been reached. During the production procedure, the fibrin monomer in question has been admixed a coagulation-stopping agent with a pH-value of 4.6. The restoration of fibrin monomer to the neutral state implies that the coagulation process starts again.

The device in question can also be used for other components beyond fibrin I and pH 10-buffer, where the mixing ratios and the volume flows must be adapted to these components. For example, fibrinogen-containing components and components containing thrombin or a thrombin-like enzyme can also be applied using the instant device.

## Claims

1. A system for applying a fibrin sealant to a site in need thereof, the system comprising
a spray pen (1,2) adapted to be held and actuated in one hand and comprising at one end a spray head (1) from which one or more components of said sealant and a gas are dispensable and separate channel means (5,6,7) for transferring said one or more components and gas to said spray head(1) from a second end of said pen (1,2);
tubing means (11,12,13) providing fluid communication between said second end of said spray pen (1,2) and a source of said one or more components and gas;
an expelling means (15) remote from said spray pen (1,2) comprising a source of said gas (16) and sources of said one or more components and controllable means (25) for transferring said gas and one or more components from said expelling means (15), through said tubing means (11,12,13), through said spray pen (1,2) and out of said spray head (1); and **characterised by**
actuating means located on the spray pen (1,2) in the form of a button which upon actuation causes fibrin sealant to be applied to the desired site.

2. The system of claim 1 **characterised in that** said components are a fibrin monomer solution and a component which when mixed with said solution will cause said fibrin monomer to polymerise.

3. The system of claim 1 **characterised in that** said components are a fibrinogen-containing composition and a composition containing thrombin or a thrombin-like enzyme.

4. The system of claim 1 **characterised in that** a control means for controlling the flow of said gas and components is located on said spray pen (1,2) and is capable of communicating with said expelling means (15) via signal means.

5. A system as claimed in claim 1 **characterised in that** the spray head is provided with a separate outlet channel (5,6,7) for each component as well as a separate outlet channel for gas fed through a gas-transferring means (13) from a gas source (15), said outlets (8,9,10) of the component outlet channels (5,6,7) and the gas outlet channel in the head (1) being aligned along a substantially rectilinear line, the outlet (10) of the gas outlet channel being accommodated at the end of said line of outlets (8,9,10) and wherein the spray head (1) is integral with an elongated handle (2) and wherein the component and gas-transferring means are substantially resilient tubings (11,12,13) extending from the handle (2) to a remote location of the expelling means (15).

6. A system as claimed in claim 5 **characterised in that** said gas outlet (10) is at one end of said straight line of outlets (8,9,10).

7. A system as claimed in claim 5 **characterised in that** said components form a fibrin sealant upon said application to said desired site.

8. A system as claimed in claim 5 **characterised in that** an actuating means (24) is provided in connection with the handle (2), which actuating means (24) actuates the expelling means (15) and the feeding of gas through a differential pressure-registering device.

9. A system as claimed in claim 5 **characterised in that** the containers used for the components are syringes (19) with their respective pistons for expelling said components, further wherein the expelling means (15) comprises a holder for the securing of the syringes and an electrically driven elevator means (20) for a simultaneous actuation of the pistons of the syringes (19).

10. A system as claimed in claim 5 **characterised in that** the expelling means (15) and the gas source in the form of a gas pump (16) are combined as a separate unit together with an electrically driven control unit (25), which is adapted to control the expelling means (15) and the gas pump (16) at a signal from the user of the spray head (1).

## Patentansprüche

1. System zur Verabreichung eines Fibrinversiegelungsmittels an eine versiegelungsbedürftige Stelle, wobei das System aufweist:
einen Sprühstift (1, 2), der in einer Hand gehalten und betätigt werden kann und der aufweist: an einem Ende einen Sprühkopf (1), aus dem eine oder mehrere Komponenten des Versiegelungsmittels und ein Gas abgegeben werden können, und getrennte Kanaleinrichtungen (5, 6, 7) zum Überführen der einen oder mehreren Komponenten und des Gases von einem zweiten Ende des Stifts (1, 2) an den Sprühkopf (1);
Schlauchleitungseinrichtungen (11, 12, 13), die Fluidkommunikation zwischen dem zweiten Ende des Sprühstifts (1, 2) und einer Quelle der einen oder mehreren Komponenten und des Gases ermöglichen;
eine Ausstoßeinrichtung (15), die von dem Sprühstift (1, 2) entfernt ist, mit einer Quelle des Gases (16) und Quellen der einen oder mehreren Komponenten und einer steuerbaren Einrichtung (25) zum Überführen des Gases und der einen oder mehreren Komponenten aus der Ausstoßeinrichtung (15) durch die Schlauchleitungseinrichtungen (11, 12, 13), durch den Sprühstift (1, 2) und aus dem Sprühkopf (1) hinaus; und **gekennzeichnet durch**
eine Betätigungseinrichtung, die am Sprühstift (1, 2) in Form eines Knopfes angeordnet ist, der bei Betätigung bewirkt, daß Fibrinversiegelungsmittel an die gewünschte Stelle verabreicht wird.

2. System nach Anspruch 1, **dadurch gekennzeichnet, daß** die Komponenten eine Fibrinmonomerlösung und eine Komponente sind, die, wenn sie mit der Lösung gemischt wird, bewirkt, daß das Fibrinmonomer polymerisiert.

3. System nach Anspruch 1, **dadurch gekennzeichnet, daß** die Komponenten eine fibrinogenhaltige Verbindung und eine Verbindung sind, die Thrombin oder ein thrombinartiges Enzym enthält.

4. System nach Anspruch 1, **dadurch gekennzeichnet, daß** eine Steuereinrichtung zur Steuerung des Durchflusses des Gases und der Komponenten am Sprühstift (1, 2) angeordnet ist und in der Lage ist, mit der Ausstoßeinrichtung (15) über eine Signaleinrichtung zu kommunizieren.

5. System nach Anspruch 1, **dadurch gekennzeichnet, daß** der Sprühkopf mit einem getrennten Auslaßkanal (5, 6, 7) für jede Komponente sowie mit einem getrennten Auslaßkanal für das Gas versehen ist, das durch eine Gasüberleitungsseinrichtung (13) von einer Gasquelle (15) zugeführt wird, wobei die Auslässe (8, 9, 10) der Komponentenauslaßkanäle (5, 6, 7) und des Gasauslaßkanals im Kopf (1) entlang einer im wesentlichen geraden Linie ausgerichtet sind, wobei der Auslaß (10) des Gasauslaßkanals am Ende der Linie der Auslässe (8, 9, 10) untergebracht ist und wobei der Sprühkopf (1) mit einem langgestreckten Griff (2) einstückig ist und wobei die Komponentenund Gasüberleitungseinrichtungen im wesentlichen elastische Schlauchleitungen (11, 12, 13) sind, die sich vom Griff (2) bis zu einer entfernten Stelle der Ausstoßeinrichtung (15) erstrecken.

6. System nach Anspruch 5, **dadurch gekennzeichnet, daß** der Gasauslaß (10) an einem Ende der geraden Linie der Auslässe (8, 9, 10) ist.

7. System nach Anspruch 5, **dadurch gekennzeichnet, daß** die Komponenten nach der Verabreichung an die gewünschte Stelle ein Fibrinversiegelungsmittel bilden.

8. System nach Anspruch 5, **dadurch gekennzeichnet, daß** eine Betätigungseinrichtung (24) in Verbindung mit dem Griff (2) vorgesehen ist, wobei die Betätigungseinrichtung (24) die Ausstoßeinrichtung (15) betätigt und die Zuführung des Gases durch eine Differenzdruckregistriervorrichtung bewirkt.

9. System nach Anspruch 5, **dadurch gekennzeichnet, daß** die Behälter, die für die Komponenten verwendet werden, Spritzen (19) mit jeweiligen Kolben zum Ausstoßen der Komponenten sind, wobei die Ausstoßeinrichtung (15) ferner einen Halter zur festen Anordnung der Spritzen und eine elektrisch betriebene Anhebe-Einrichtung (20) für eine gleichzeitige Betätigung der Kolben der Spritzen (19) aufweist.

10. System nach Anspruch 5, **dadurch gekennzeichnet, daß** die Ausstoßeinrichtung (15) und die Gasquelle in Form einer Gaspumpe (16) als eine gesonderte Einheit zusammen mit einer elektrisch betriebenen Steuereinheit (25) kombiniert sind, die geeignet ist, die Ausstoßeinrichtung (15) und die Gaspumpe (16) bei einem Signal vom Benutzer des Sprühkopfs (1) zu steuern.

## Revendications

1. Système pour appliquer un matériau d'obturation à base de fibrine sur un site en présentant le besoin, le système comprenant
un crayon de pulvérisation (1, 2) conçu pour être tenu et actionné d'une main et comprenant à une extrémité une tête de pulvérisation (1) hors de laquelle un ou plus composants dudit matériau d'obturation et un gaz peuvent être distribués et des moyens de canaux séparés (5,6, 7) pour transférer ledit un ou plus composants et le gaz à ladite tête de pulvérisation (1) hors de la seconde extrémité dudit crayon (1,2) ;
des moyens de tuyaux flexibles (11 à 13) assurant une communication fluidique entre ladite seconde extrémité dudit crayon de pulvérisation (5,2) et une source dudit un ou plus composants et du gaz ;
un moyen d'expulsion (15) éloignés dudit crayon du pulvérisation (1,2) comprenant une source dudit gaz (16) et des sources dudit un ou plus composants et un moyen pouvant être commandé (25) pour transférer ledit gaz et lesdits un ou plus composants hors dudit moyen d'expulsion (15), à travers lesdits moyens de tuyaux flexibles (11,12,13), à travers ledit crayon de pulvérisation (1,2) et hors de ladite tête de pulvérisation (un) ; et **caractérisé par**
un moyen d'actionnement situé sur le crayon de pulvérisation (1,2) sous la forme d'un bouton qui, lors d'un actionnement, amène le matériau d'obturation à base de fibrine à être appliqué sur le site désiré.

2. Système selon la revendication 1, **caractérisé en ce que** lesdits composants sont une solution de monomère de fibrine et un composant qui, lorsqu'il est mélangé à ladite solution, amènera ledit monomère de fibrine à polymériser.

3. Système selon la revendication 1, **caractérisé en ce que** lesdits composants sont une composition contenant un fibrinogène et une composition contenant de la thrombine ou une enzyme du type thrombine.

4. Système selon la revendication 1, **caractérisé en ce qu'**un moyen de commande pour réguler le flux dudit gaz et desdits composants est situé sur ledit crayon de pulvérisation (1,2) et est apte à communiquer avec ledit moyen d'expulsion (15) via un moyen générateur de signal.

5. Système selon la revendication 1, **caractérisé en ce que** la tête de pulvérisation est munie d'un canal de sortie séparé (5,6,7) pour chaque composant, de même qu'un canal de sortie séparé pour le gaz amené à travers un moyen de transfert de gaz (13) à partir d'une source de gaz (15), lesdites sorties (8,9,10) des canaux de sortie des composants et le canal de sortie de gaz dans la tête à buses (1) étant alignés en une rangée sensiblement rectiligne, la sortie (10) du canal de sortie de gaz étant formée à l'extrémité de ladite rangée de sorties (8,9,10) et dans lequel les moyens de transfert de composants et de gaz sont des tuyaux flexibles appréciablement résilients (11,12,13) s'étendant de la poignée (2) à un emplacement éloigné du moyen d'expulsion (15).

6. Système selon la revendication 5, **caractérisé en ce que** ladite sortie de gaz (10) est située à une extrémité de ladite rangée rectiligne de sorties (8,9,10).

7. Système selon la revendication 5, **caractérisé en ce que** lesdits composants forment un matériau d'obturation à base de fibrine lors de ladite application sur ledit site désiré.

8. Système selon la revendication 5, **caractérisé en ce qu'**un moyen d'actionnement (24) est fourni en liaison avec la poignée (2), lequel moyen d'actionnement (24) actionne le moyen d'expulsion (15) et l'amenée de gaz par l'intermédiaire d'un dispositif de régulation de pression différentielle.

9. Système selon la revendication 5, **caractérisé en ce que** les conteneurs utilisés pour les composants sont des seringues (19) munies de leur piston respectif pour expulser lesdits composants, en outre dans lequel le moyen d'expulsion (15) comprend un support pour fixation des seringues et un moyen élévateur mû électriquement (20) pour un actionnement simultané des pistons des seringues (19).

10. Système selon la revendication 5, **caractérisé en ce que** le moyen d'expulsion (15) et la source de gaz, sous la forme d'une pompe à gaz (16), sont combinés en une unité séparée associée à une unité de commande mue électriquement (25), laquelle est conçue pour commander le moyen d'expulsion (15) et la pompe à gaz (16) sur un signal provenant de l'utilisateur de la tête de pulvérisation (1).
